Europäisches Patentamt

European Patent Office

Office européen des brevets

(11) Publication number: **0 199 872**
**B1**

(12) **EUROPEAN PATENT SPECIFICATION**

(45) Date of publication of patent specification: 25.04.90

(51) Int. Cl.⁵: **A 61 N 2/08**

(21) Application number: **85305103.5**

(22) Date of filing: **17.07.85**

(54) Magnetic therapeutic instrument.

(30) Priority: **14.03.85 JP 37355/85**

(43) Date of publication of application:
**05.11.86 Bulletin 86/45**

(45) Publication of the grant of the patent:
**25.04.90 Bulletin 90/17**

(84) Designated Contracting States:
**DE FR GB IT NL**

(56) References cited:
**EP-A-0 036 022**
**DE-A-2 235 015**
**DE-A-2 806 088**
**DE-A-2 914 904**
**FR-A-2 460 660**

(73) Proprietor: **Okubo, Masao**
**167-10, Aza Nogami**
**Kuchitanaka, Amagasaki (JP)**

(72) Inventor: **Okubo, Masao**
**167-10, Aza Nogami**
**Kuchitanaka, Amagasaki (JP)**

(74) Representative: **Beresford, Keith Denis Lewis**
**et al**
**BERESFORD & Co. 2-5 Warwick Court High**
**Holborn**
**London WC1R 5DJ (GB)**

Courier Press, Leamington Spa, England.

## Description

This invention relates to a magnetic therapeutic instrument and, in particular, to a magnetic therapeutic instrument exhibiting therapeutic effects produced by a magnetic field and germanium as well as stimulative effect on the human skin by pressing.

Recently, various kinds of medical instruments utilizing the action of germanium have been proposed for the purpose of allaying pain. A chip of germanium stuck to the affected part of the human body can significantly allay the pain in the body. Effects of allaying such pains as stiffness in the shoulder, whiplash, lumbago, gout, and arthritis have been reported.

Germanium, however, is highly expensive and accordingly a therapeutic instrument using it is inclined to be costly.

On the other hand, magnetic therapeutic instruments of a type provided with a permanent magnet embedded into a foundation sheet made of wood or synthetic resin or of a type consisting of adhesive plaster containing magnetic powder have been proposed.

There is known from DE-A-2806088 and US-A-4162672 a magnetic therapeutic instrument having a magnet of 500 to 1000 Oe (about 40 to 80 kAm$^{-1}$) in the form of a ferrite disc. One side of the ferrite disc contacts an adhesive base sheet and the other side contacts the skin. The side contacting the skin may be the N or the S pole of the magnet. A paper release sheet is affixed to the surface of the adhesive base sheet except at the magnet.

The abovesaid two kinds of known instruments are devoid of action to cause electric current to flow directly through the human body, thereby therapeutic effect thereof being limited.

The Applicant has found that germanium in the form of a thin film rather than a solid chip can easily permeate the human body and DC current, though weak, can be caused to flow through the human body via metallic tapes of an instrument fabricated in accordance with this invention.

Accordingly, the present invention provides a magnetic therapeutic instrument as set out in the claims.

Thus, a magnetic therapeutic instrument is provided capable of utilising the combined therapeutic effects provided by germanium and magnetism.

The magnetic therapeutic instrument may be relatively inexpensive.

It appears that a magnetic therapeutic instrument embodying the present invention may cause a weak electric current to flow directly through the human body.

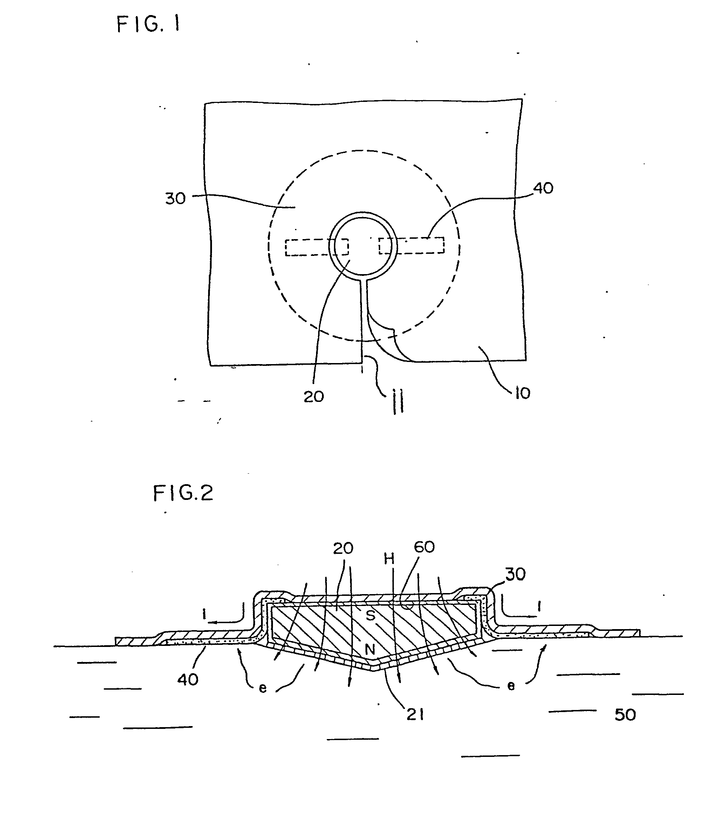

One embodiment of the invention will now be described by way of example only, with reference to Figures 1 and 2 of the accompanying drawings in which:

Figure 1 is a plan view of a magnetic therapeutic instrument embodying this invention; and,

Figure 2 is a representation of the above instrument applied to the affected part on the human skin.

In the drawing, the reference numeral 10 indicates a detachable thick sheet which may be made of material of any kind (e.g. paper) but must be capable of sticking to and readily being detached from the adhesive foundation material that will be described later.

This detachable paper sheet is provided with an opening slightly larger in diameter than the permanent magnet 20 as shown in the drawing and a cut part 11 communicating with the abovesaid opening for convenience of removing the sheet from the magnet. Therefore, the abovesaid detachable paper sheet 10 is stuck to the part of the surface of an adhesive foundation material 30 other than the part thereof to which the permanent magnet is stuck.

The adhesive foundation material 30 is circular or the like in shape, for example, an adhesive plaster, to which the reverse surface of permanent magnet 20 made of, for example, ferrite is stuck.

The permanent magnet 20 is a ferrite magnet in the shape of a flattened spinning top and magnetized so as to be N and S in polarity at the front and the reverse surfaces, respectively, and to range from 750 to 850 oersteds (about 60 to 68 kAm$^{-1}$) in intensity of magnetic field. (However, the magnet may be in the shape of coin). As a result, a magnetic field H is formed as shown in Figure 2. The entire surface of the permanent magnet is coated with gold or other precious metal 60 (for example, silver) in thickness of several microns by means of plating or evaporation. The surface to be brought into contact with the human skin is coated with a germanium layer 21 as shown in Figure 2.

Coating of precious metal 60 over the surface of the ferrite magnet as described above is intended for forming a circuit for causing electric current to flow through the human skin via metallic tapes welded to the magnet. The permanent magnet is not limited to ferrite type and may be of other rare-earth type.

The germanium layer 21 is 0.05-30 μm in thickness and, is preferably formed by the cluster ion beam method in a state such that the C-axis direction of the film is aligned with the axis of the permanent magnet 20.

The numeral 40 indicates a tape made of, for example, gold, copper or other metallic material and having one end electrically connected to the reverse surface of the permanent magnet 20 by means of, for example, welding. A single piece of metallic tape may suffice, however, two pieces arranged symmetrically with respect to the permanent magnet as shown in the drawing or more pieces may also fulfil the purpose.

In applying such a magnetic therapeutic instrument as above to the human body, after removing the detachable paper sheet 10, the permanent magnet 20 is pressed so that the surface thereof is in tight contact with the affected part 50 of the human body and the adhesive foundation material 30 is also stuck to the skin. The metallic tapes 40 remain stuck to the adhesive foundation material 30 when the detachable paper sheet 10 is removed and, therefore, can easily be stuck to the affected part.

The inventor has found that weak electric current ranging from 0.01 to 1 µA flows through the skin as described above with the use of a magnetic therapeutic instrument as illustrated. DC current is indicated as i which flows in the direction from the reverse surface of the permanent magnet 20 to the human skin as shown in Figure 2, whereby the flow of electrons e is verified to be present within the skin as shown therein.

The effect of a magnetic therapeutic instrument embodying this invention was as follows:

With the application of a magnetic therapeutic instrument as illustrated to 200 male adults of ages from 25 to 65 who were suffering from stiffness in their shoulders, 150 were relieved from the pain (group A), 30 felt themselves relieved slightly but not so distinctly (B), and remaining 20 felt that the therapy was ineffective (C).

On the other hand, in the case of application of the instrument having no metallic tape, the numbers of persons in the groups A, B and C were 52, 13, and 135, respectively.

Such investigation as above repeated several times on other patients proved that the magnetic therapeutic instrument of this invention provided with metallic tapes which are essential requirement for fabrication of this instrument is superior to other instruments in therapeutic effect.

Further, a trial application of a modification of such instruments as above magnetized to be S and N in polarity at the front and the reverse surfaces thereof (metallic tapes were stuck to the magnet) indicated that the numbers of persons in the groups A, B, and C were 32, 28, and 60, respectively. That is to say, the polarity of the permanent magnet is an important factor for the structure of the instrument of this invention and the front surface (to be applied to the human skin) should be N in polarity as well as the reverse surface be S for the best effect.

The illustrated magnetic therapeutic instrument can be used repeatedly. However, it has been found that the germanium layer coating the permanent magnet disappears with repeated use of the instrument as often as 5 times. It is presumed that permeation of germanium molecules into the human skin accounts for the disappearance of the germanium layer.

## Claims

1. A magnetic therapeutic instrument comprising:
    a permanent magnet (20) having polarities of N and S on a front surface and a reverse surface, respectively;
    an adhesive base material (30) adhered to the reverse surface of said permanent magnet; and a detachable sheet (10) adhered to a part of the front surface of said adhesive base material so as not to cover said permanent magnet, characterised in that the permanent magnet (20) has a magnetic field of 60-68 kAm$^{-1}$ in strength and is entirely coated with precious metal (60), in that at least one metallic tape (40) is electrically connected at one end to the reverse surface of said permanent magnet (20), and in that the front surface of said permanent magnet is coated, above the precious metal layer, with a germanium layer (21) 0.05-30 µm thick with its C-axis direction aligned with the axis of the field of said permanent magnet.

2. A magnetic therapeutic instrument as set forth in claim 1, wherein said permanent magnet (20) is shaped approximately like a coin.

3. A magnetic therapeutic instrument as set forth in claim 1, wherein said permanent magnet (20) has the shape of a flattened spinning top.

4. A magnetic therapeutic instrument having a surface (21) capable of being impressed against the skin (50) of a mammal, and means (20) for generating a magnetic field through said surface (21), characterised in that the surface (21) is a germanium surface, in that the means (20) for generating a magnetic field generates a north pole at the germanium surface, and in that the instrument further comprises means (40) for establishing a current path from said germanium surface (21) to said skin (50) in a direction transverse to said magnetic field.

5. A magnetic therapeutic instrument as claimed in claim 4, wherein the C-axis of said germanium (21) is substantially perpendicular to said germanium surface (21).

6. A magnetic therapeutic instrument as claimed in claim 4, wherein the C-axis of said germanium (21) is substantially parallel to the axis of the magnetic field.

7. A magnetic therapeutic instrument as claimed in any of claims 4 to 6, wherein said germanium surface is the surface of a thin germanium film (21).

8. A magnetic therapeutic instrument as claimed in any of claims 4 to 7, wherein said means for establishing a current path comprises one or more metallic tapes (40).

9. A magnetic therapeutic instrument as claimed in claim 8 as dependant on claim 7, wherein said means for generating a magnetic field comprises a body (20) of magnetic or magnetisable material, and wherein a layer (60) of inert metal is deposited on said body, said germanium film (26) is deposited on said layer of inert metal, and said metallic tape (40) is electrically connected to said layer of inert metal.

## Patentansprüche

1. Magnetisches Therapieinstrument, umfassend:
    einen Permanentmagneten (20), der einen Nordpol an einer Vorderfläche und einen Südpol an einer Hinterfläche hat;

ein klebendes Basismaterial (30), welches an der Hinterfläche des Permanentmagneten anhaften gelassen wird; und

ein abnehmbares Blatt (10), welches an einem Teil der Vorderfläche des klebenden Basismaterials derart anhaften gelassen wird, daß es den permanentmagneten nicht bedeckt, dadurch gekennzeichnet, daß der permanentmagnet (20) ein Magnetfeld einer Stärke von 60 bis 68 kAm$^{-1}$ hat und vollständig mit Edelmetall (60) überzogen bzw. beschichtet ist, daß wenigstens ein Metallband (40) an einem Ende mit der Hinterfläche des Permanentmagneten (20) elektrisch verbunden ist, und daß die Vorderfläche des Permanentmagneten über der Edelmetallschicht mit einer Germaniumschicht (21) einer Dicke von 0,05 bis 30 µm beschichtet ist, deren C-Achsenrichtung mit der Achse des Feldes des Permanentmagneten ausgerichtet ist.

2. Magnetisches Therapieinstrument nach Anspruch 1, wobei der Permanentmagnet (20) annähernd wie eine Münze gestaltet ist.

3. Magnetisches Therapieinstrument nach Anspruch 1, wobei der Permanentmagnet (20) die Gestalt einer flachen Drehspitze hat.

4. Magnetisches Therapieinstrument, welches eine Fläche (21), die gegen die Haut (50) eines Säugers gedrückt werden kann, und eine Einrichtung (20) hat zum Erzeugen eines Magnetfeldes durch die genannte Fläche (21), dadurch gekennzeichnet, daß die Fläche (21) eine Germaniumfläche ist, daß die Einrichtung (20) zum Erzeugen eines Magnetfeldes einen Nordpol an der Germaniumfläche erzeugt, und daß das Instrument weiterhin Mittel (40) aufweist zum Bilden eines Stromweges von der Germaniumfläche (21) zu der Haut (50) in einer Richtung quer zu dem Magnetfeld.

5. Magnetisches Therapieinstrument nach Anspruch 4, wobei die C-Achse des Germaniums (21) im wesentlichen rechtwinklig zu der Germaniumfläche (21) verläuft.

6. Magnetisches Therapieinstrument nach Anspruch 4, wobei die C-Achse des Germaniums (21) im wesentlichen parallel zur Achse des Magnetfeldes verläuft.

7. Magnetisches Therapieinstrument nach irgendeinem der Ansprüche 4 bis 6, wobei die Germaniumfläche die Fläche eines dünnen Germaniumfilmes bzw. einer dünnen Germaniumfolie (21) ist.

8. Magnetisches Therapieinstrument nach irgendeinem der Ansprüche 4 bis 7, wobei die Mittel zum Bilden eines Stromweges ein Metallband oder mehrere Metallbänder (40) umfassen.

9. Magnetisches Therapieinstrument nach Anspruch 8, wenn abhängig von Anspruch 7, wobei die Einrichtung zum Erzeugen eines Magnetfeldes einen Körper (20) aus magnetischem oder magnetisierbarem Material aufweist, und wobei eine Schicht (60) aus Inertmetall an dem Körper angelagert ist, wobei der Germaniumfilm bzw. die Germaniumfolie (21) an der Schicht aus Inertmetall angelagert ist und das Metallband (40) mit der Schicht aus Inertmetall elektrisch verbunden ist.

## Revendications

1. Instrument thérapeutique magnetique comprenant:

un aimant permanent (20) présentant des polarités N et S respectivement sur sa surface avant et sur sa surface arrière;

un matériau de base adhésif (30) collé à la surface arrière de l'aimant permanent; et une feuille détachable (10) collée à une partie de la surface avant du matériau de base adhésif, de manière à ne pas recouvrir l'aimant permanent,

instrument caractérisé en ce que l'aimant permanent (20) présente un champ magnétique d'une force de 750 à 850 oersteds (environ 60 à 68 kAm$^{-1}$), et se trouve entièrement recouvert de métal précieux (60), en ce qu'au moins un ruban métallique (40) est relié électriquement par une extrémité à la surface arrière de l'aiment permanent (20), et en ce que la surface avant de cet aimant permanent est recouverte, au-dessus de la couche de métal précieux, d'une couche de germanium (21) de 0,05 à 30 µm d'épaisseur de façon que sa direction d'axe C soit alignée sur l'axe du champ magnétique de l'aimant permanent.

2. Instrument thérapeutique magnétique selon la revendication 1, caractérisé en ce que l'aimant permanent (20) est réalisé sensiblement en forme de pièce de monnaie.

3. Instrument thérapeutique magnétique selon la revendication 1, caractérisé en ce que l'aimant permanent (20) a la forme d'une toupie aplatie.

4. Instrument thérapeutique magnétique comportant une surface (21) capable d'être appuyée contre la peau (50) d'un être humain ou d'un mammifère, et des moyens (20) destinés à produire un champ magnétique à travers cette surface (21), instrument caractérisé en ce que la surface (21) est une surface de germanium, en ce que les moyens (20) pour produire un champ magnétique produisent un pôle nord à l'endroit de la surface de germanium, et en ce que l'instrument comprend, en outre, des moyens (40), pour établir un circuit de courant, entre la surface de germanium (21) et la peau (50) dans une direction transversale par rapport au champ magnétique.

5. Instrument thérapeutique magnétique selon la revendication 4, caractérisé en ce que l'axe C du germanium (21) est sensiblement perpendiculaire à cette surface de germanium (21).

6. Instrument thérapeutique magnétique selon la revendication 4, caractérisé en ce que l'axe C du germanium (21) est sensiblement parallèle à l'axe du champ magnétique.

7. Instrument thérapeutique magnétique selon l'une quelconque des revendications 4 à 6, caractérisé en ce que la surface de germanium est la surface d'un film mince de germanium (21).

8. Instrument thérapeutique magnétique selon l'une quelconque des revendications 4 à 7, caractérisé en ce que les moyens pour établir un circuit de courant comprennent un ou plusieurs rubans métalliques (40).

9. Instrument thérapeutique magnétique selon la revendication 8 dépendant de la revendication 7, caractérisé en ce que les moyens, pour produire un champ magnétique, comprennent un corps (20) en matériau magnétique ou magnétisable, et en ce qu'une couche (60) de métal inerte est déposée sur ce corps, le fil de germanium (26) étant déposé sur cette couche de métal inerte et le ruban métallique (40) étant relié électriquement à cette couche de métal inerte.

FIG. 1

FIG.2